# EUROPEAN PATENT SPECIFICATION

(11) **EP 3 117 850 B1**
(45) Date of publication and mention of the grant of the patent: **08.05.2019**
(21) Application number: 16178446.7
(22) Date of filing: 07.07.2016
(51) Int. Cl.: A61M 5/00

(54) **APPLICATOR WITH PLUNGER HAVING MOVABLE RANGE RESTRICTED BY RESISTANCE STRUCTURE**
APPLIKATOR MIT KOLBEN MIT DURCH EINE WIDERSTANDSSTRUKTUR BEGRENZTER REICHWEITE
APPLICATEUR AVEC PISTON AYANT UNE PLAGE MOBILE RESTREINTE PAR LA STRUCTURE DE RÉSISTANCE

(30) Priority: 14.07.2015 JP 2015140832
(43) Date of publication of application: 18.01.2017
(73) Proprietor: Shofu Inc., Kyoto-shi, Kyoto (JP)
(72) Inventor: TAKEI, Ryouji, Soka-shi, Saitama (JP); YONEDA, Akira, Soka-shi,, Saitama (JP); SAKAMOTO, Shuji, Kyoto-shi,, Kyoto (JP); KADOBAYASHI, Yusei, Kyoto-shi,, Kyoto (JP)
(74) Representative: Mewburn Ellis LLP

(56) References cited:
- US-A1- 2002 045 865
- US-A1- 2006 178 644
- US-A1- 2010 198 164
- US-B1- 6 579 269
- US-B2- 8 953 977

## Description

### Technical Field

The present invention relates to an applicator that includes a cylindrical syringe including a cartridge fitting portion provided at one of two ends of the cylindrical syringe and configured to be fitted with a cartridge, and a plunger configured to be inserted into the cylindrical syringe and to press a piston inside the cartridge.

### Background Art

An example applicator 200, which includes a cylindrical syringe and a plunger, is disclosed in Fig. 15 of JP 2003-79642 A. The cylindrical syringe 210 has two ends, and includes a cartridge fitting portion at one of the two ends of the cylindrical syringe 210. The cartridge fitting portion is configured to be fitted with a cartridge 212 which includes a cylindrical portion 216 having two ends and an opening portion formed at one of the two ends of the cylindrical portion 216, a nozzle portion 218 provided at the other of the two ends of the cylindrical portion 216, and a piston 74 (Fig. 11 of JP 2003-79642 A) disposed inside the cylindrical portion 216 to push out a content contained in the cylindrical portion 216 from the nozzle portion 218. The plunger has two ends, and includes a pressing portion provided at one of the two ends of the plunger and configured to be inserted into the cylindrical syringe 210 from the other of the two ends of the cylindrical syringe 210 and to press the piston 74 inside the cartridge 212 fitted with the cartridge fitting portion, and an operated portion extending through the other end of the cylindrical syringe 210 and configured to be pressed by a finger of an operator.

Another example applicator, which includes a cylindrical syringe 526 and a plunger 26, is disclosed in Fig. 16 of JP 2001-112782 A. The cylindrical syringe 526 includes a cartridge fitting portion 527 configured to be fitted with a cartridge 210 which does not include a piston and the body of which is deformable to push out a content contained in the cartridge 210 from a nozzle 218. The plunger 26 is configured to be inserted into the cylindrical syringe 526, and includes an operated portion 42 extending through one of two ends of the cylindrical syringe 526 opposite to the cartridge fitting portion 527 and configured to be pressed by a finger of an operator.
US2006/178644 describes a syringe with a cartridge having a central piston.

### Summary of Invention

### Technical Problem

Judging from the respective configurations of the applicators disclosed in JP 2003-79642 A and JP 2001-112782 A, it is considered that the cylindrical syringe is made of metal and that the plunger is made of metal although not clearly stated in the documents. The applicators made of metal advantageously have a high mechanical strength and can be repeatedly used, but are too expensive to be disposable. If the applicators are so expensive, expanded sales of the cartridges may be adversely affected.

The present invention is defined in the appended claims. However, the description and drawings shall be used to interpret the appended claims.

An object of the present invention is to provide an applicator that is molded from a resin material and that is inexpensive compared to those according to the related art.

Another object of the present invention is to provide an applicator in which a plunger does not easily slip out of a cylindrical syringe.

### Solution to Problem

An applicator according to the present invention includes a cylindrical syringe molded from a resin material and a plunger molded from a rein. The cylindrical syringe has two ends, and includes a cartridge fitting portion at one of the two ends of the cylindrical syringe. The cartridge fitting portion is configured to be fitted with a cartridge which includes a cylindrical portion having two ends and an opening portion formed at one of the two ends of the cylindrical portion, a nozzle portion provided at the other of the two ends of the cylindrical portion, and a piston disposed inside the cylindrical portion to push out a content contained in the cylindrical portion from the nozzle portion. The plunger has two ends and includes a pressing portion provided at one of the two ends of the plunger and configured to be inserted into the cylindrical syringe from the other of the two ends of the cylindrical syringe and to press the piston inside the cartridge fitted with the cartridge fitting portion, and an operated portion provided at the other of the two ends of the plunger, extending through the other end of the cylindrical syringe, and configured to be pressed by a finger of an operator. Thus, the price of the applicator can be significantly reduced compared to that according to the related art. In the present invention, a resistance structure is provided between an inner peripheral surface of the cylindrical syringe and an outer peripheral surface of the pressing portion of the plunger. The resistance structure is configured to act as resistance to movement of the pressing portion when the pressing portion is moved toward the cartridge fitting portion beyond a predetermined position and to movement of the pressing portion when the pressing portion is moved from the side of the cartridge fitting portion toward the other end of the cylindrical syringe beyond the predetermined position. The resistance structure is configured to allow the pressing portion to be moved beyond the predetermined position against the resistance when a force equal to or greater than a predetermined force is applied to the plunger in a longitudinal direction of the plunger. It is difficult to provide resin molded products with a high precision compared to metallic parts because of expansion and contraction of a resin. Further, the plunger inserted into the cylindrical syringe may easily slip out of the cylindrical syringe. For these reasons, without the resistance structure described above, the plunger may slip out of the cylindrical syringe when a user uses the applicator with the plunger being directed downward and releases his/her finger from the operated portion of the plunger. Therefore, when an applicator without the resistance structure is used, it is necessary for the user to always support the operated portion of the plunger with his/her finger so that the plunger does not slip off by itself. In the present invention, the resistance structure is provided between the inner peripheral surface of the cylindrical syringe and the outer peripheral surface of the pressing portion of the plunger. Thus, even though the cylindrical syringe and the plunger are molded from a resin material, the plunger does not easily slip out of the cylindrical syringe unless a force equal to or greater than a predetermined force is applied to the plunger in the longitudinal direction of the plunger. Thus, according to the present invention, it is possible to provide a practically trouble-free applicator at a low price even if the cylindrical syringe and the plunger are molded from a resin material. This allows the applicator to be disposable.

The resistance structure may be located at any position between the cartridge fitting portion of the cylindrical syringe and the other end of the cylindrical syringe.

The resistance structure may include at least one outer projecting portion integrally formed with the inner peripheral surface of the cylindrical syringe to project radially inward, and at least one inner projecting portion integrally formed with the outer peripheral surface of the pressing portion of the plunger to project radially outward. In this configuration, the resin material for forming the cylindrical syringe and a shape of the at least one outer projecting portion and the resin material for forming the plunger and a shape of the at least one inner projecting portion may be determined such that the at least one inner projecting portion and the at least one outer projecting portion are deformed to allow the at least one inner projecting portion to pass over the at least one outer projecting portion when a force equal to or greater than the predetermined force is applied to the plunger. With this configuration, the at least one inner projecting portion and the at least one outer projecting portion, which are components required for the resistance structure, can be integrally formed with the cylindrical syringe and the plunger, respectively, when the cylindrical syringe and the plunger are molded.

Preferably, the resin material for forming the cylindrical syringe and the resin material for forming the plunger are polypropylene. Polypropylene is available at a low unit price, has a high chemical resistance, has high strength and elasticity compared to polyethylene, and therefore is suitably used to mold the cylindrical syringe and the plunger.

The at least one inner projecting portion may be constituted from one annular projecting portion that continuously annularly extends on the outer peripheral surface of the pressing portion of the plunger, and a cross-sectional shape of the annular projecting portion may have a profile of which the height gradually increases continuously toward an apex of the profile, as the annular projecting portion is cut in a direction orthogonal to a circumferential direction of the plunger. In this case, preferably, the at least one outer projecting portion is constituted from a plurality of distributed projecting portions provided at predetermined angular intervals in a circumferential direction of the cylindrical syringe. A cross-sectional shape of each distributed projecting portion has a profile of which the height gradually increases continuously toward an apex of the profile, as the distributed projecting portions are cut in a direction orthogonal to the circumferential direction of the cylindrical syringe and a cross-sectional shape of each distributed projecting portion has a profile of which the height gradually increases continuously toward an apex of the profile, as the distributed projecting portions are cut in the circumferential direction of the cylindrical syringe.

Conversely, the at least one outer projecting portion may be constituted from one annular projecting portion that continuously annularly extends on the inner peripheral surface of the cylindrical syringe, and a cross-sectional shape of the annular projecting portion may have a profile of which the height gradually increases continuously toward an apex of the profile, as the annular projecting portion is cut in a direction orthogonal to a circumferential direction of the cylindrical syringe. In this case, preferably, the at least one inner projecting portion is constituted from a plurality of distributed projecting portions provided at predetermined angular intervals in a circumferential direction of the plunger. A cross-sectional shape of each distributed projecting portion has a profile of which the height gradually increases continuously toward an apex of the profile, as the distributed projecting portions are cut in the direction orthogonal to the circumferential direction of the plunger, and a cross-sectional shape of each distributed projecting portion has a profile of which the height gradually increases continuously toward an apex of the profile, as the distributed projecting portions are cut in the circumferential direction of the plunger.

In such configuration as the resistance structure is constituted from the annular projecting portion and the plurality of distributed projecting portions, the annular projecting portion and the plurality of distributed projecting portions can be easily deformed when a force equal to or greater than a predetermined force is applied to the plunger in the longitudinal direction of the plunger when either one of the annular projecting portion and the plurality of distributed projecting portions passes over the other. In contrast, after either one of the annular projecting portion and the plurality of distributed projecting portions has passed over the other, either one of the annular projecting portion and the plurality of distributed projecting portions will not pass over the other in the opposite direction even if the plunger is rotated in the cylindrical syringe unless a predetermined force is applied to the plunger in the longitudinal direction of the plunger. Thus, the plunger can be prevented from slipping off with a simple structure.

Preferably, the profile of each distributed projecting portion has a flat portion provided at the apex of the profile, and a skirt formed on the side of the cartridge fitting portion with respect to the flat portion is longer than a skirt formed on the side of the other end of the cylindrical syringe with respect to the flat portion. In such configuration as the distributed projecting portions include the flat portion, the apexes of the distributed projecting portions are more uniformly worn when either one of the annular projecting portion and the plurality of distributed projecting portions passes over the other. This prevents the apexes of the distributed projecting portions from being worn out early even if an action of either one of the annular projecting portion and the plurality of distributed projecting portions passing over the other or a converse action is repeatedly performed a certain number of times. As a result, the applicator can be used a plurality of times even if the cylindrical syringe and the plunger are molded from a resin material. In such configuration as the profile of each distributed projecting portion has the flat portion, production thereof can more accurately be managed.

The predetermined angular intervals between the plurality of distributed projecting portions are arbitrary, but are preferably 60°, 90°, or 120°. With such an angle, both high molding precision and high strength can be easily achieved even if the cylindrical syringe and the plunger are molded from a resin material.

The resistance structure may be disposed at an arbitrary position. Preferably, however, the resistance structure is provided adjacent to the cartridge fitting portion of the cylindrical syringe. With this positional arrangement, the distance by which the plunger is moved downward by itself from the cylindrical syringe when the operator holds the applicator with the plunger being directed downward can be reduced, thereby improving the operability of the applicator.

Preferably, a stopper portion is provided in the vicinity of the operated portion of the plunger to abut on the other end of the cylindrical syringe. With such stopper portion, it is possible to prevent the operator from pushing the plunger too far into the cylindrical syringe to cause the pressing portion located at the leading end of the plunger molded from a resin material to apply a strong force to the cartridge fitted with the cartridge fitting portion and thereby to break the cartridge and the cartridge fitting portion.

Preferably, the plunger is shaped such that the outer peripheral surface of a portion that is adjacent to the stopper portion is proximate to the inner peripheral surface of the cylindrical syringe. With such structure, the plunger can be stably moved along the cylindrical syringe at the final stage of pushing in the plunger. As a result, the operability of the applicator is improved. In the state in which the plunger has been pushed in, the plunger can be suppressed from moving in the direction of slipping out of the cylindrical syringe, thereby facilitating handling of the applicator.

Preferably, the resistance structure is positioned such that the cartridge can be fitted with the cartridge fitting portion and the cartridge can be removed from the cartridge fitting portion when the at least one inner projecting portion of the plunger is in contact with the at least one outer projecting portion. With this configuration, the cartridge can be mounted and removed without removing the plunger.

### Brief Description of Drawings

Figs. 1A to 1F are a front view, a bottom view, a plan view, a left side view, a right side view, and a perspective view, respectively, illustrating that an applicator is mounted with a cartridge.
Figs. 2A to 2D are a bottom view, a right side view, a left side view, and a perspective view, respectively, of a cylindrical syringe.
Figs. 3A to 3D are a front view, a left side view, a right side view, and a perspective view, respectively, of a plunger.
Fig. 4A is a vertical sectional view illustrating the applicator mounted with the cartridge when the plunger is withdrawn; and Fig. 4B is a vertical sectional view illustrating the applicator mounted with the cartridge when the plunger is pushed in.
Fig. 5A is an enlarged sectional view illustrating a main portion of the applicator mounted with the cartridge when the plunger is withdrawn; an upper part of Fig. 5B is an enlarged view illustrating the shape of a distributed projecting portion; and a lower part of Fig. 5B is a sectional view as taken along line C-C of the upper part of Fig. 5B.
Fig. 6 is an enlarged vertical sectional view illustrating a main portion of the applicator mounted with the cartridge when the plunger is pushed in.
Fig. 7A is a front view of the cartridge; and Fig. 7B is a vertical sectional view of the cartridge.
Fig. 8 is an enlarged vertical sectional view of a nozzle portion of the cartridge.
Figs. 9A and 9B are a vertical sectional view and a horizontal sectional view, respectively of the cartridge; and Fig. 9C is a sectional view illustrating a main portion of the applicator including a piston.
Figs. 10A to 10C illustrate how the cartridge is mounted to the applicator.

### Description of Embodiments

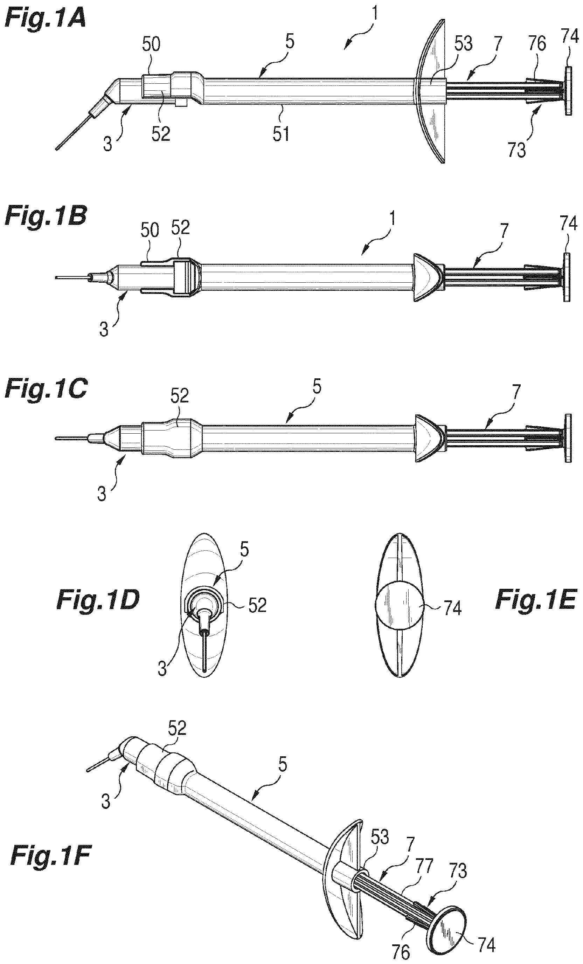

Now, with reference to the accompanying drawings, an embodiment of the present invention will be described below in detail, wherein the present invention is applied to an applicator used for discharging a paste-type dental material. Figs. 1A to 1F are a front view, a bottom view, a plan view, a left side view, a right side view, and a perspective view, respectively, illustrating an applicator 1 of the present embodiment mounted with a cartridge 3. The applicator 1 includes a cylindrical syringe 5 and a plunger 7. In Figs. 1A-1C and 1F, the plunger 7 is located at a stand-by position as discussed later.

### <Applicator>

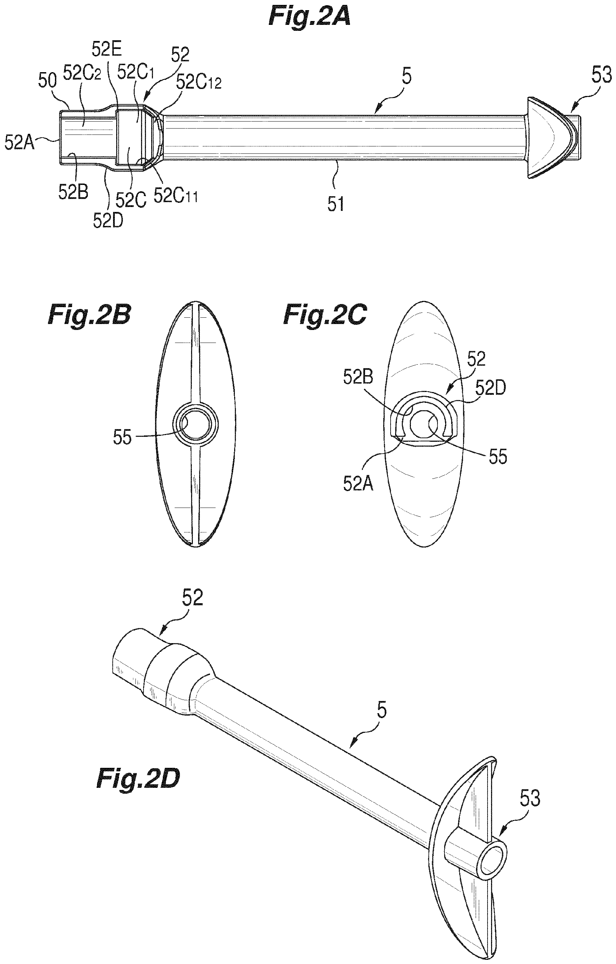

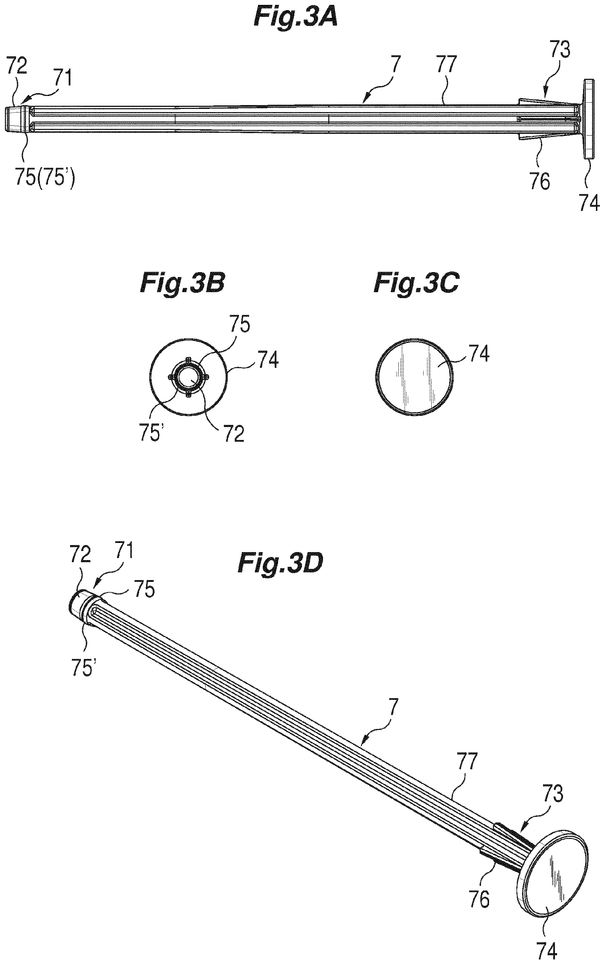

The applicator 1 includes the cylindrical syringe 5 and the plunger 7. As illustrated in Figs. 1A-1F and 2A-2D, the cylindrical syringe 5 is integrally molded from a resin material such as polypropylene or polyethylene, and has two ends. The cylindrical syringe 5 includes a cartridge fitting portion 52 provided at one 50 of the two ends of the cylindrical syringe 5 and configured to be fitted with the cartridge 3. As illustrated in Figs. 1A-1F and 3A-3D, the plunger 7 is integrally molded from a resin material such as polypropylene or polyethylene, and has two ends. The plunger 7 includes a pressing portion 72 provided at one 71 of the two ends of the plunger 7 and configured to be inserted into the cylindrical syringe 5 from the other 53 of the two ends of the cylindrical syringe 5 and to press a piston 4 inside the cartridge 3 fitted with the cartridge fitting portion 52, and an operated portion 74 provided at the other 73 of the two ends of the plunger 7, extending through the other end 53 of the cylindrical syringe 5, and configured to be pressed by a finger of an operator. The cylindrical syringe 5 and the plunger 7 are each molded integrally from a resin material, which makes it possible to significantly reduce the price of the applicator 1 compared to the conventional ones.

In the present embodiment, a stopper portion 76 that abuts on the other end 53 of the cylindrical syringe 5 is provided in the vicinity of the operated portion 74 of the plunger 7. Providing such stopper portion 76 in this way prevents the operator from pushing the plunger 7 too far into the cylindrical syringe 5 to break the plunger 7 or the cylindrical syringe 5 which is made of a resin material. The plunger 7 is shaped such that the outer peripheral surface of a portion 77 that is adjacent to the stopper portion 76 is proximate to the inner peripheral surface of the cylindrical syringe 5. With adoption of such structure, the plunger 7 can be stably moved along the cylindrical syringe 5 when the plunger 7 is pushed in. As a result, the plunger 7 is not swung when the plunger 7 is pushed in, thereby improving the operability of the applicator 1. In addition, the plunger 7 advantageously does not easily slip out of the cylindrical syringe 5.

### <Resistance structure>

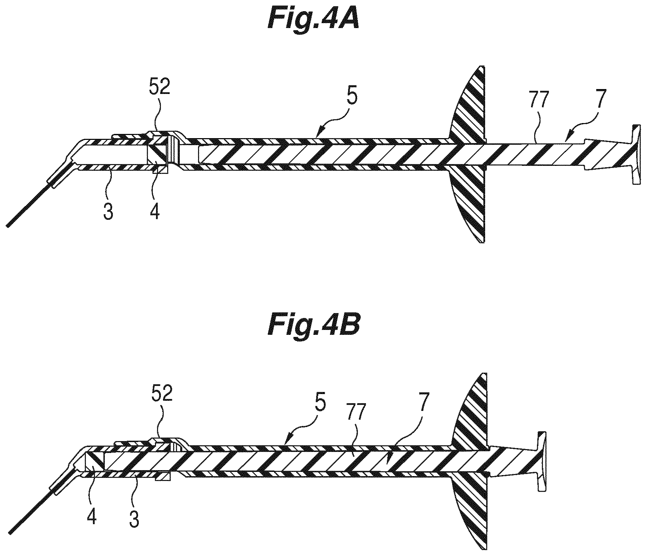

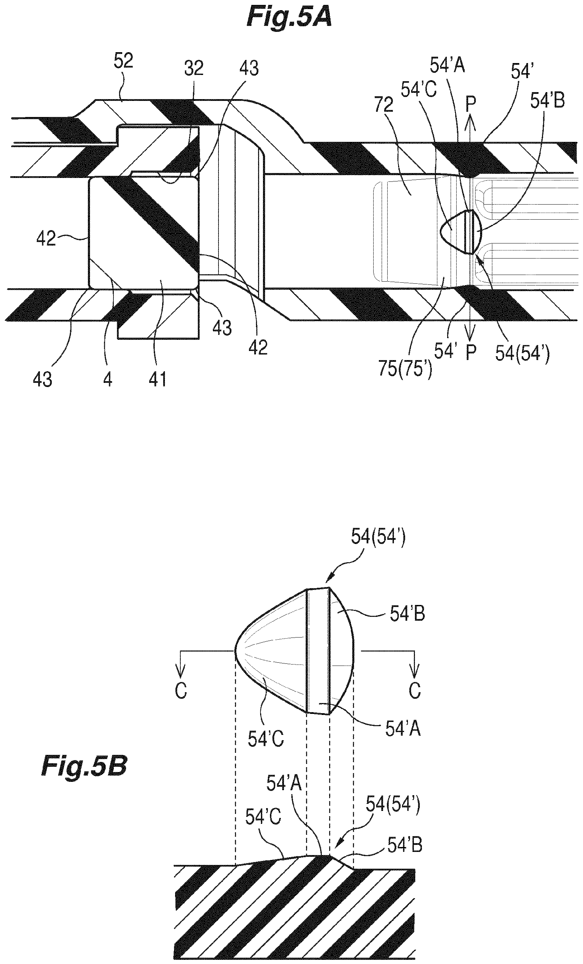

In the present embodiment, as illustrated in Figs. 4A-4B, 5A-5B, and 6, a resistance structure (54, 75) is provided between the inner peripheral surface of the cylindrical syringe 5 and the outer peripheral surface of the pressing portion 72 of the plunger 7. The resistance structure (54, 75) acts as resistance to movement of the pressing portion 72 when the pressing portion 72 is moved toward the cartridge fitting portion 52 beyond a predetermined position P shown in Fig. 5A and to movement of the pressing portion 72 when the pressing portion 72 is moved from the side of the cartridge fitting portion 52 toward the other end 53 of the cylindrical syringe 5 beyond the predetermined position P. The resistance structure (54, 75) is configured to allow the pressing portion 72 to be moved beyond the predetermined position P against the resistance when a force equal to or greater than a predetermined force is applied to the plunger 7 in the longitudinal direction of the plunger 7.

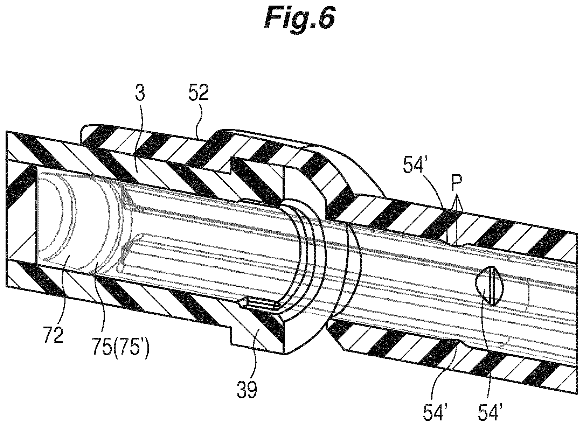

In the embodiment, as illustrated enlargedly in Figs. 5A and 6, the resistance structure (54, 75) includes at least one outer projecting portion 54 integrally formed with the inner peripheral surface of the cylindrical syringe 5 to project radially inward, and at least one inner projecting portion 75 integrally formed with the outer peripheral surface of the pressing portion 72 of the plunger 7 to project radially outward. The resin material for forming the cylindrical syringe 5 and the shape of the at least one outer projecting portion 54 and the resin material for forming the plunger 7 and the shape of the at least one inner projecting portion 75 are determined such that the at least one inner projecting portion 75 and the at least one outer projecting portion 54 are deformed to allow the at least one inner projecting portion 75 to pass over the at least one outer projecting portion 54 when a force equal to or greater than the predetermined force is applied to the plunger 7. The resin materials are preferably polypropylene. With this configuration, the inner projecting portion 75 and the outer projecting portion 54, which are constituents required for the resistance structure (54, 75), can be integrally formed with the cylindrical syringe 5 and the plunger 7, respectively, when the cylindrical syringe 5 and the plunger 7 are molded.

As illustrated in Figs. 3A-3D, 5A, and 6, the at least one inner projecting portion 75 provided on the plunger 7 is constituted from one annular projecting portion 75' that continuously annularly extends on the outer peripheral surface of the pressing portion 72 of the plunger 7, and the cross-sectional shape of the annular projecting portion 75' has a mountain-like profile of which the height gradually increases continuously toward the apex of the profile, as the annular projecting portion 75' is cut in a direction orthogonal to the circumferential direction of the plunger 7. The at least one outer projecting portion 54 is constituted from four distributed projecting portions 54' provided at predetermined angular intervals in the circumferential direction. The cross-sectional shape of each distributed projecting portion 54' has a profile of which the height gradually increases continuously toward the apex of the profile, as the distributed projecting portions 54' are cut in a direction orthogonal to the circumferential direction of the cylindrical syringe 5. The cross-sectional shape of each distributed projecting portion 54' has a profile of which the height gradually increases continuously toward the apex of the profile, as the distributed projecting portions 54' are cut in the circumferential direction of the cylindrical syringe 5. The predetermined angular intervals between the plurality of distributed projecting portions 54' may be determined as desired, but are preferably 60°, 90°, or 120°. With these angles, both high molding precision and high strength can be easily achieved even if the cylindrical syringe 5 and the plunger 7 are molded from a resin material.

As illustrated enlargedly in Fig. 5B, the profile of each distributed projecting portion 54' has a flat portion 54'A provided at the apex of the profile, and a skirt 54'C formed on the side of the cartridge fitting portion 52 [see Fig. 1A] with respect to the flat portion 54'A is longer than a skirt 54'B formed on the side of the other end 53 [see Fig. 1A] of the cylindrical syringe 5 with respect to the flat portion 54'A. If the distributed projecting portions 54' include the flat portion 54'A, the apexes of the distributed projecting portions 54' are more uniformly worn when either one of the annular projecting portion 75' and the four distributed projecting portions 54' passes over the other. This prevents the apexes of the distributed projecting portions 54' from being worn out early even if an action of either one of the annular projecting portion and the plurality of distributed projecting portions passing over the other or a converse action is repeatedly performed a certain number of times . As a result, the applicator 1 can be used a plurality of times even if the cylindrical syringe 5 and the plunger 7 are molded from a resin material. Providing the flat portion 54'A on each distributed projecting portion 54' can increase the processing precision of the distributed projecting portion 54'.

In contrast, the resistance structure according to the embodiment described above, the at least one outer projecting portion 54 may be constituted from one annular projecting portion that continuously annularly extends on the inner peripheral surface of the cylindrical syringe 5, and the cross-sectional shape of the annular projecting portion may have a mountain-like profile of which the height gradually increases continuously toward the apex of the profile, as the annular projecting portion is cut in a direction orthogonal to the circumferential direction of the cylindrical syringe 5. In this configuration, the at least one inner projecting portion 75 may be constituted from a plurality of distributed projecting portions provided at predetermined angular intervals in the circumferential direction of the plunger 7, and the cross-sectional shape of each distributed projecting portion may have a profile of which the height gradually increases continuously toward the apex of the profile, as the distributed projecting portions are cut in the direction orthogonal to the circumferential direction of the plunger 7 and the cross-sectional shape of each distributed projecting portion may have a profile of which the height gradually increases continuously toward the apex of the profile, as the distributed projecting portions are cut in the circumferential direction of the plunger 7.

In a configuration as in the present embodiment, wherein the resistance structure (54, 75) is constituted from the one annular projecting portion 75' and the plurality of distributed projecting portions 54', the annular projecting portion 75' and the plurality of distributed projecting portions 54' can be easily deformed when a force equal to or greater than a predetermined force is applied to the plunger 7 in the longitudinal direction of the plunger 7 as either one of the annular projecting portion 75' and the plurality of distributed projecting portions 54' passes over the other. In contrast, after either one of the annular projecting portion 75' and the plurality of distributed projecting portions 54' has passed over the other, either one of the annular projecting portion 75' and the plurality of distributed projecting portions 54' does not pass over the other in the opposite direction, even if the plunger 7 is rotated in the cylindrical syringe 5, unless a predetermined force is applied to the plunger 7 in the longitudinal direction of the plunger 7. Thus, the plunger 7 can be prevented from slipping off with a simple structure.

The position P of the resistance structure (54, 75) may be determined as desired. Preferably however, the resistance structure is provided adjacent to the cartridge fitting portion 52 of the cylindrical syringe 5 as in the present embodiment. If the resistance structure is located at the position P, it is possible to reduce the distance by which the plunger 7 is moved downward from the cylindrical syringe 5 when the operator holds the applicator 1 with the plunger 7 being directed downward, thereby improving the operability of the applicator 1. In practice, the resistance structure (54, 75) is preferably positioned such that the cartridge 3 can be fitted with the cartridge fitting portion 52 and the cartridge 3 can be removed from the cartridge fitting portion 52 when the at least one inner projecting portion 75 of the plunger 7 is in contact with the at least one outer projecting portion 54. With this configuration, the cartridge 3 can be mounted and removed without removing the plunger 7.

### <Cartridge>

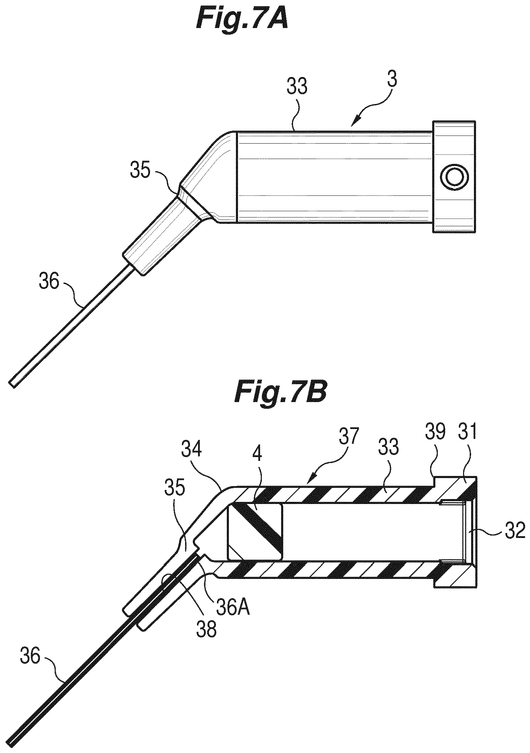

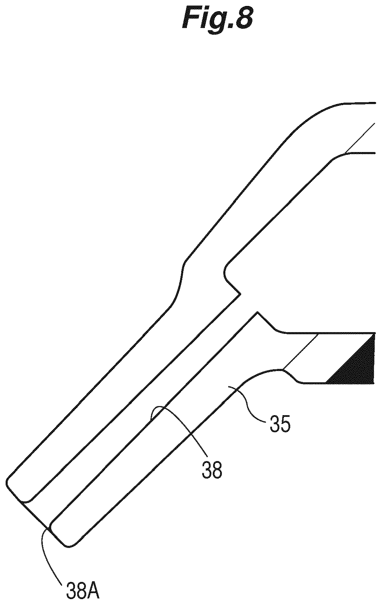

As illustrated in Figs. 7A and 7B, the cartridge 3 includes a cylindrical portion 33 having two ends and an opening portion 32 and a flange portion 39 formed at one 31 of the two ends of the cylindrical portion 33, a nozzle portion 35 provided at the other 34 of the two ends of the cylindrical portion 33, and the piston 4 disposed inside the cylindrical portion 33 to push out a content contained in the cylindrical portion 33 from the nozzle portion 35. The nozzle portion 35 includes, as a nozzle, a pipe 36 made of metal and provided at the other end 34 of the cylindrical portion 33. The cylindrical portion 33 and the nozzle portion 35 are integrally molded from a resin material such as polypropylene. Preferably, an entire surface of the end portion 36A of the pipe 36 is curved such that no angled portion is present. With this configuration, it is possible to prevent the inner wall of a through hole 38 from being shaved by the pipe 36 when inserting the pipe 36 into the through hole 38, thereby furthermore preventing the swarf from being produced and pushed out as mixed in the content. As illustrated in Fig. 8, an annular tapered surface 38A is formed at an entrance portion of the through hole 38, and the tapered surface 38A becomes larger in radial dimension toward an opening end surface of the entrance portion. The tapered surface 38A is formed not to contact the outer peripheral surface of the pipe 36 when the pipe 36 is press-fitted into the through hole 38. Such a tapered surface 38A facilitates insertion of the pipe 36 into the through hole 38.

The through hole 38 is shaped such that the radial dimension of the through hole 38, which extends in the longitudinal direction of the nozzle portion 35 continuously with the tapered surface 38A, becomes gradually smaller and thereafter constant. With this configuration, the pipe 36 can be easily inserted to the middle of the through hole 38, after which the pipe 36 is press-fitted, thereby alleviating the workload of press-fitting.

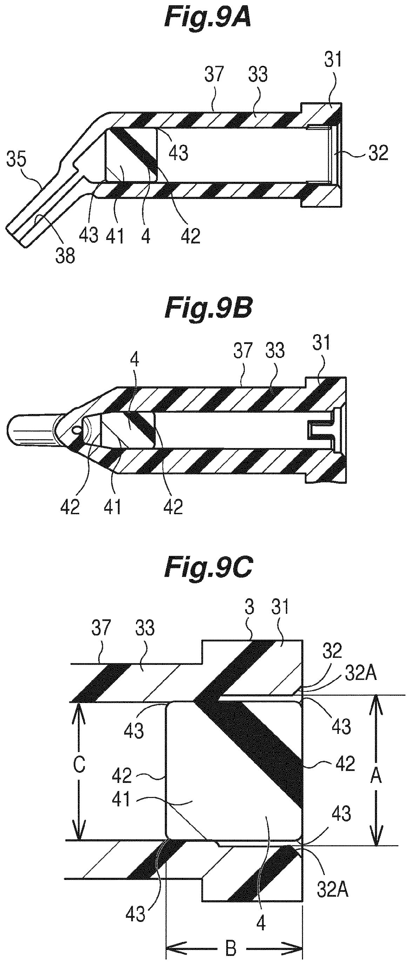

The piston 4 is integrally molded from a resin material such as polypropylene. As illustrated in Figs. 9A to 9C, a pair of piston-side tapered surfaces 43 or piston-side curved surfaces 43 are formed between an annular peripheral wall surface 41 of the piston 4 and a pair of circular end surfaces 42, 42 of the piston 4 located on both sides in the thickness direction of the piston 4. The pair of piston-side tapered surfaces 43 or piston-side curved surfaces 43 become smaller in radial dimension from the annular peripheral wall surface 41 toward the pair of circular end surfaces 42, 42. In Figs. 9A and 9B, one of the end portions of the piston 4 has been deformed to conform to the shape of the inner wall surface of the cylindrical portion 33 of a cartridge body 37.

As illustrated in Fig. 9C, a body-side tapered surface 32A or a body-side curved surface 32A is formed at the opening portion 32 at the one end of the cylindrical portion 33 of the cartridge body 37, and the body-side tapered surface 32A or the body-side curved surface 32A becomes smaller in radial dimension toward the other end of the cylindrical portion 33. The inner angle of the body-side tapered surface 32A or the radius of curvature of the body-side curved surface 32A is larger than the inner angle of the pair of piston-side tapered surfaces 43 or the radius of curvature of the pair of piston-side curved surfaces 43 formed on the piston 4. This configuration further facilitates insertion of the piston 4 from the opening portion 32 of the cylindrical portion 33.

### <Structure of cartridge fitting portion>

As illustrated in Figs. 2A and 2B, the cartridge fitting portion 52 configured to be fitted with the cartridge 3 includes an end surface opening portion 52A opened in an extending direction in which a cylindrical body 51 of the cylindrical syringe 5 extends, a continuous opening portion 52B continuous with the end surface opening portion 52A, opened in a radial direction, and extending in the extending direction, and a recess portion 52C configured to communicate with the continuous opening portion 52B and an internal passage 55 in the cylindrical body 51. The recess portion 52C includes a flange fitting recess 52C1 to be fitted with the flange portion 39 of the cartridge 3, and a cylindrical portion fitting recess 52C2 configured to communicate with the flange fitting recess 52C1 and to be fitted with a part of the cylindrical portion 33. A wall portion 52D surrounding the cylindrical portion fitting recess 52C2 of the cartridge fitting portion 52 is configured to warp into snap engagement with the part of the cylindrical portion 33 when the part of the cylindrical portion 33 is inserted from the continuous opening portion 52B. Specifically, a sectional shape of an inner wall surface of the wall portion 52D surrounding the cylindrical portion fitting recess 52C2 has an arcuate profile that is continuous with the continuous opening portion 52B, as the wall portion 52D is cut in a direction orthogonal to a longitudinal direction of the cylindrical syringe 5, and the arcuate profile has an arcuate angle larger than 180 degrees. With such a structure, a sufficient force for holding the cylindrical portion 33, which is required for snap engagement, can be secured. The term "snap engagement" refers to engagement obtained when the wall portion 52D surrounding the cylindrical portion fitting recess 52C2 holds the cylindrical portion 33 utilizing a force generated in the wall portion 52D which has been deformed to be opened and then is going to return to its original state. The flange fitting recess 52C1 includes a first portion 52C11 to be fitted with the flange portion 39, and a second portion 52C12 located between the flange portion 39 and the cylindrical body 51 when the first portion 52C11 is fitted with the flange portion 39. In the present embodiment, the second portion 52C12 is shaped to become gradually smaller in radial dimension as the second portion 52C12 extends toward the cylindrical body 51. The wording "(to be) shaped to become gradually smaller in radial dimension" may mean in other words that the inner wall surface of a wall portion surrounding the second portion 52C12 of the flange fitting recess 52C1 constitutes a part of a conical surface having its apex located on the side of the cylindrical body 51.

A stepped portion 52E to be engaged with the flange portion 39 is formed between the flange fitting recess 52C1 and the cylindrical portion fitting recess 52C2. The stepped portion 52E is configured to entirely contact the end surface of the flange portion 39 located on the side of the cylindrical portion 33. With adoption of this structure, the flange portion 39 is pressed against the stepped portion 52E when the piston 4 is pushed by the plunger 7, thereby preventing the cartridge 3 from popping out of the cartridge fitting portion 52.

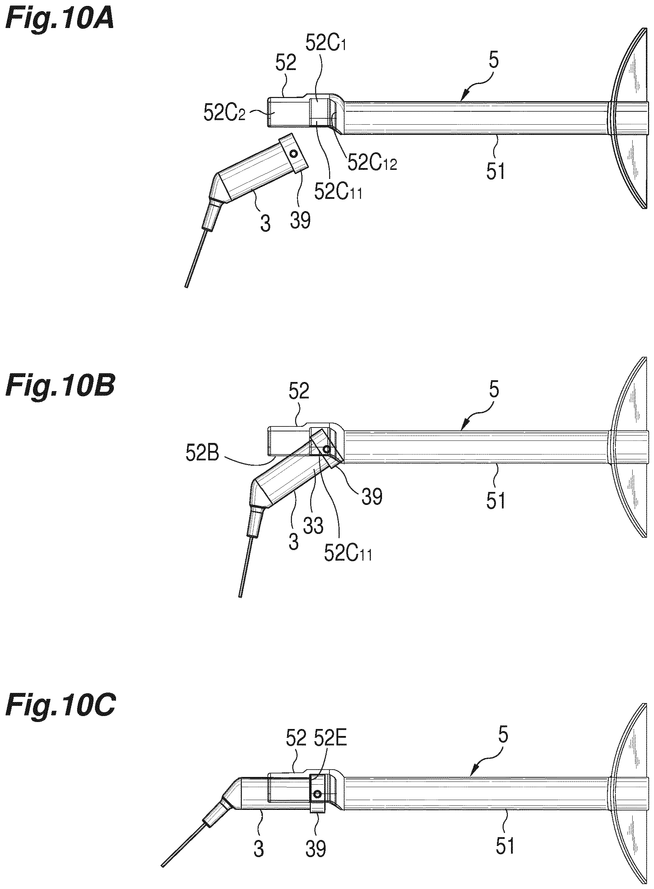

When mounting the cartridge 3, as illustrated in Figs. 10A and 10B, the cylindrical portion 33 is pushed into the cylindrical portion fitting recess 52C2 through the continuous opening portion 52B while inserting the flange portion 39 of the cartridge 3 into the first portion 52C11 of the flange fitting recess 52C1 in order to achieve snap engagement. When removing the cartridge 3, the cylindrical portion 33 is extracted from the cylindrical portion fitting recess 52C2 by causing the distal end side of the cylindrical portion 33 to get out from the continuous opening portion 52B while lifting up the cylindrical portion 33 using the flange portion 39 as the fulcrum, and thereafter the flange portion 39 is extracted from the flange fitting recess 52C1. During this operation, the flange portion 39 can be tilted while sliding on the inner wall surface of a wall portion surrounding the second portion 52C12 of the flange fitting recess 52C1 since the second portion 52C12 is shaped to become gradually smaller in radial dimension as the second portion 52C12 extends toward the cylindrical body 51. As a result, the cartridge 3 can be easily removed using the flange portion 39 as the fulcrum. The cartridge fitting portion 52 of such structure can easily be integrally formed with the cylindrical syringe 5. As a result, it is possible to provide an applicator made of a resin material with ease and at a low price.

While the preferred embodiments of the present invention have been described and shown herein, the present invention should not be construed in a limiting sense. It should be understood that various modifications, rearrangements, and substitutions may be made without departing from the scope of the present invention.

### Industrial Applicability

In a configuration according to the present invention wherein the resistance structure is provided between the inner peripheral surface of the cylindrical syringe and the outer peripheral surface of the pressing portion of the plunger, the plunger will not readily slip off from the cylindrical syringe, even if the cylindrical syringe and the plunger are each molded from a resin material, unless a force equal to or greater than a predetermined force is applied to the plunger in the longitudinal direction of the plunger. According to the present invention, therefore, a practically trouble-free applicator can be provided at low cost even though the cylindrical syringe and the plunger are molded from a resin material, thereby enabling the applicator to be disposable.

## Claims

1. An applicator comprising:
a cylindrical syringe (5) molded from a resin material and having two ends, the cylindrical syringe (5) including a cartridge fitting portion (52) at one (50) of the two ends of the cylindrical syringe (5), the cartridge fitting portion (52) being configured to be fitted with a cartridge (3) which includes a cylindrical portion (33) having two ends and an opening portion (32) formed at one (31) of the two ends of the cylindrical portion (33), a nozzle portion (35) provided at the other (34) of the two ends of the cylindrical portion (33), and a piston (4) disposed inside the cylindrical portion (33) to push out a content contained in the cylindrical portion (33) from the nozzle portion (35); and
a plunger (7) molded from a resin material and having two ends, the plunger (7) including a pressing portion (72) provided at one (71) of the two ends of the plunger (7) and configured to be inserted into the cylindrical syringe (5) from the other (53) of the two ends of the cylindrical syringe (5) and to press the piston (4) inside the cartridge (3) fitted with the cartridge fitting portion (52), and an operated portion (74) provided at the other (73) of the two ends of the plunger (7), extending through the other end (53) of the cylindrical syringe (5), and configured to be pressed by a finger of an operator, **characterized in that**:
the applicator further comprises a resistance structure (54, 75) provided between an inner peripheral surface of the cylindrical syringe (5) and an outer peripheral surface of the pressing portion (72) of the plunger (7), the resistance structure being configured to act as resistance to movement of the pressing portion (72) when the pressing portion (72) is moved toward the cartridge fitting portion (52) beyond a predetermined position (P) and to movement of the pressing portion (72) when the pressing portion (72) is moved from the side of the cartridge fitting portion (52) toward the other end (53) of the cylindrical syringe (5) beyond the predetermined position (P), wherein
the resistance structure (54, 75) is configured to allow the pressing portion (72) to be moved beyond the predetermined position (P) against the resistance when a force equal to or greater than a predetermined force is applied to the plunger (7) in a longitudinal direction of the plunger (7).

2. The applicator according to claim 1, wherein:
the resistance structure (54,75) includes at least one outer projecting portion (54) integrally formed with the inner peripheral surface of the cylindrical syringe (5) to project radially inward, and at least one inner projecting portion (75) integrally formed with the outer peripheral surface of the pressing portion (72) of the plunger (7) to project radially outward; and
the resin material for forming the cylindrical syringe (5) and a shape of the at least one outer projecting portion (54) and the resin material for forming the plunger (7) and a shape of the at least one inner projecting portion (75) are determined such that the at least one inner projecting portion (75) and the at least one outer projecting portion (54) are deformed to allow the at least one inner projecting portion (75) to pass over the at least one outer projecting portion (54) when a force equal to or greater than the predetermined force is applied to the plunger (7) .

3. The applicator according to claim 2, wherein
the resin material for forming the cylindrical syringe (5) and the resin material for forming the plunger (7) are polypropylene.

4. The applicator according to claim 2 or 3, wherein:
the at least one inner projecting portion (75) is constituted from one annular projecting portion (75') that continuously annularly extends on the outer peripheral surface of the plunger (7), and a cross-sectional shape of the annular projecting portion (75') has a profile of which the height gradually increases continuously toward an apex of the profile, as the annular projecting portion (75') is cut in a direction orthogonal to a circumferential direction of the plunger (7) ; and
the at least one outer projecting portion (54) is constituted from a plurality of distributed projecting portions (54') provided at predetermined angular intervals in a circumferential direction of the cylindrical syringe (5), and a cross-sectional shape of each distributed projecting portion (54') has a profile of which the height gradually increases continuously toward an apex of the profile, as the distributed projecting portions (54') are cut in a direction orthogonal to the circumferential direction of the cylindrical syringe (5) and a cross-sectional shape of each distributed projecting portion (54') has a profile of which the height gradually increases continuously toward an apex of the profile, as the distributed projecting portions (54') are cut in the circumferential direction of the cylindrical syringe (5).

5. The applicator according to claim 2 or 3, wherein:
the at least one outer projecting portion is constituted from one annular projecting portion that continuously annularly extends on the inner peripheral surface of the cylindrical syringe (5), and a cross-sectional shape of the annular projecting portion has a profile of which the height gradually increases continuously toward an apex of the profile, as the annular projecting portion is cut in a direction orthogonal to a circumferential direction of the cylindrical syringe (5); and
the at least one inner projecting portion is constituted from a plurality of distributed projecting portions provided at predetermined angular intervals in a circumferential direction of the plunger (7), and a cross-sectional shape of each distributed projecting portion has a profile of which the height gradually increases continuously toward an apex of the profile, as the distributed projecting portions are cut in the direction orthogonal to the circumferential direction of the plunger (7) and a cross-sectional shape of each distributed projecting portion has a profile of which the height gradually increases continuously toward an apex of the profile, as the distributed projecting portions are cut in the circumferential direction of the plunger (7).

6. The applicator according to claim 4 or 5, wherein
the profile of each distributed projecting (54') portion has a flat portion (54'A) provided at the apex of the profile, and a skirt (54'C) formed on the side of the cartridge fitting portion (52) with respect to the flat portion (54'A) is longer than a skirt (54'B) formed on the side of the other end (53) of the cylindrical syringe (5) with respect to the flat portion (54'A).

7. The applicator according to claim 6, wherein
the predetermined angular intervals are intervals of 60°, 90°, or 120°.

8. The applicator according to claim 1, wherein
the resistance structure (54,75) is provided adjacent to the cartridge fitting portion (52) of the cylindrical syringe (5) .

9. The applicator according to claim 1, wherein
a stopper portion (76) is provided in the vicinity of the operated portion (74) of the plunger (7) to abut on the other end (53) of the cylindrical syringe (5).

10. The applicator according to claim 9, wherein
the plunger (7) is shaped such that the outer peripheral surface of a portion (77) that is adjacent to the stopper portion (76) is proximate to the inner peripheral surface of the cylindrical syringe (5).

11. The applicator according to claim 2, wherein
the resistance structure (54,75) is positioned such that the cartridge (3) can be fitted with the cartridge fitting portion (52) and the cartridge (3) can be removed from the cartridge fitting portion (52) when the at least one inner projecting portion (75) of the plunger (7) is in contact with the at least one outer projecting portion (54) of the cylindrical syringe (5).

## Patentansprüche

1. Applikator, der Folgendes umfasst:
eine zylinderförmige Spritze (5), die aus einem Harzmaterial geformt ist und zwei Enden aufweist, wobei die zylinderförmige Spritze (5) einen Kartuscheinsetzabschnitt (52) an einem (50) der beiden Enden der zylinderförmigen Spritze (5) aufweist, wobei der Kartuscheinsetzabschnitt (52) ausgebildet ist, dass eine Kartusche (3) eingesetzt wird, die einen zylinderförmigen Abschnitt (33) mit zwei Enden und einem Öffnungsabschnitt (32) umfasst, der an einem (31) der beiden Enden des zylinderförmigen Abschnitts (33) ausgebildet ist, einen Düsenabschnitt (35) am anderen Ende (34) der beiden Enden des zylinderförmigen Abschnitts (33) bereitgestellt ist und einen Kolben (4) umfasst, der im Inneren des zylinderförmigen Abschnitts (33) angeordnet ist, um einen in dem zylinderförmigen Abschnitt (33) enthaltenen Inhalt von dem Düsenabschnitt (35) auszustoßen; und
einen Stößel (7), der aus einem Harzmaterial geformt ist und zwei Enden aufweist, wobei der Stößel (7) einen Druckabschnitt (72), der an einem (71) der beiden Enden des Stößels (7) bereitgestellt ist und ausgebildet ist, um in die zylinderförmige Spritze (5) vom anderen (53) der beiden Enden der zylinderförmigen Spritze (5) eingebracht zu werden und den Kolben (4) im Inneren der Kartusche (3), die in den Kartuscheinsetzabschnitt (52) eingesetzt ist, und einen Betätigungsabschnitt (74) umfasst, der am anderen (73) der beiden Enden des Stößels (7) bereitgestellt ist, sich durch das andere Ende (53) der zylinderförmigen Spritze (5) hindurch erstreckt und ausgebildet ist, um durch einen Finger eines Bedieners gedrückt zu werden, **dadurch gekennzeichnet, dass**:
der Applikator ferner eine Widerstandsstruktur (54, 75) umfasst, die zwischen einer Innenumfangsoberfläche der zylinderförmigen Spritze (5) und einer Außenumfangsoberfläche des Druckabschnitts (72) des Stößels bereitgestellt ist, wobei die Widerstandsstruktur ausgebildet ist, um als Widerstand gegenüber der Bewegung des Druckabschnitts (72) zu wirken, wenn der Druckabschnitt (72) von der Seite des Kartuscheinsetzabschnitts (52) in Richtung des anderen Endes (53) der zylinderförmigen Spritze (5) über die vorbestimmte Position (P) hinaus bewegt wird, wobei
die Widerstandsstruktur (54, 75) ausgebildet ist, um zu ermöglichen, dass der Druckabschnitt (72) gegen den Widerstand über die vorbestimmte Position (P) hinausbewegt wird, wenn eine Kraft auf den Stößel (7) in einer Längsrichtung des Stößels (7) angewandt wird, die einer vorbestimmten Kraft gleich oder größer als diese ist.

2. Applikator nach Anspruch 1, wobei:
die Widerstandsstruktur (54, 75) zumindest einen äußeren vorstehenden Abschnitt (54) umfasst, der einstückig mit der Innenumfangsoberfläche der zylinderförmigen Spritze (5) ausgebildet ist, um radial nach innen vorzustehen, und zumindest einen inneren vorstehenden Abschnitt (75), der einstückig mit der Außenumfangsoberfläche des Druckabschnitts (72) des Stößels (7) ausgebildet ist, um radial nach außen vorzustehen; und
das Harzmaterial zum Ausbilden der zylinderförmigen Spritze (5) und einer Form des zumindest einen äußeren vorstehenden Abschnitts (54) und das Harzmaterial zum Ausbilden des Stößels (7) und einer Form des zumindest einen inneren vorstehenden Abschnitts (75) so bestimmt werden, dass der zumindest eine innere vorstehende Abschnitt (75) und der zumindest eine äußere vorstehende Abschnitt (54) verformt werden, um zu ermöglichen, dass der zumindest eine innere vorstehende Abschnitt (75) über den zumindest einen äußeren vorstehenden Abschnitt (54) hinweg bewegt wird, wenn eine Kraft auf den Stößel (7) ausgeübt wird, die der vorbestimmten Kraft entspricht oder größer als diese ist.

3. Applikator nach Anspruch 2, wobei
das Harzmaterial zum Ausbilden der zylinderförmigen Spritze (5) und das Harzmaterial zum Ausbilden des Stößels (7) Polypropylen sind.

4. Applikator nach einem der Ansprüche 2 oder 3, wobei:
der zumindest eine innere vorstehende Abschnitt (75) durch einen ringförmigen vorstehenden Abschnitt (75') gebildet wird, der sich durchgehend ringförmig auf der Außenumfangsoberfläche des Stößels (7) erstreckt, und eine Querschnittsform des ringförmigen vorstehenden Abschnitts (75') ein Profil aufweist, dessen Höhe in Richtung eines Scheitelpunkts des Profils allmählich kontinuierlich ansteigt, wenn der ringförmige vorstehende Abschnitt (75') in einer Richtung im rechten Winkel auf eine Umfangsrichtung des Stößels (7) geschnitten wird; und
der zumindest eine äußere vorstehende Abschnitt (54) aus einer Vielzahl verteilter vorstehender Abschnitte (54') besteht, die in vorbestimmten Winkelabständen in einer Umfangsrichtung der zylinderförmigen Spritze (5) bereitgestellt sind, und eine Querschnittsform jedes der verteilten vorstehenden Abschnitte (54') ein Profil aufweist, dessen Höhe in Richtung eines Scheitelpunkts des Profils allmählich kontinuierlich ansteigt, wenn die verteilten vorstehenden Abschnitte (54') in einer Richtung im rechten Winkel auf eine Umfangsrichtung der zylinderförmigen Spritze (5) geschnitten werden, und eine Querschnittsform jedes der verteilten vorstehenden Abschnitte (54') ein Profil aufweist, dessen Höhe in Richtung eines Scheitelpunkts des Profils allmählich kontinuierlich ansteigt, wenn die verteilten vorstehenden Abschnitte (54') in Umfangsrichtung der zylinderförmigen Spritze (5) geschnitten werden.

5. Applikator nach Anspruch 2 oder 3, wobei:
der zumindest eine äußere vorstehende Abschnitt aus einem ringförmigen vorstehenden Abschnitt besteht, der sich durchgehend ringförmig auf der Innenumfangsoberfläche der zylinderförmigen Spritze (5) erstreckt, und eine Querschnittsform des ringförmigen vorstehenden Abschnitts ein Profil aufweist, dessen Höhe in Richtung eines Scheitelpunkts des Profils allmählich kontinuierlich ansteigt, wenn der ringförmige vorstehende Abschnitt in einer Richtung im rechten Winkel auf eine Umfangsrichtung der zylinderförmigen Spritze (5) geschnitten wird; und
der zumindest eine innere vorstehende Abschnitt aus einer Vielzahl verteilter vorstehender Abschnitte (54') besteht, die in vorbestimmten Winkelabständen in einer Umfangsrichtung des Stößels (7) bereitgestellt sind, und eine Querschnittsform jedes der verteilten vorstehenden Abschnitte ein Profil aufweist, dessen Höhe in Richtung eines Scheitelpunkts des Profils allmählich kontinuierlich ansteigt, wenn die verteilten vorstehenden Abschnitte in einer Richtung im rechten Winkel auf eine Umfangsrichtung des Stößels (7) geschnitten werden, und eine Querschnittsform jedes der verteilten vorstehenden Abschnitte ein Profil aufweist, dessen Höhe in Richtung eines Scheitelpunkts des Profils allmählich kontinuierlich ansteigt, wenn die verteilten vorstehenden Abschnitte in Umfangsrichtung des Stößels (7) geschnitten werden.

6. Applikator nach Anspruch 4 oder 5, wobei
das Profil jedes der verteilten vorstehenden Abschnitte (54') einen flachen Abschnitt (54'A) aufweist, der am Scheitelpunkt des Profils bereitgestellt ist, und eine Einfassung (54'C), die in Bezug auf den flachen Abschnitt (54'A)auf der Seite des Kartuscheinsetzabschnitts (52) ausgebildet ist, länger ist als eine Einfassung (54'B), die in Bezug auf den flachen Abschnitt (54'A) auf der Seite des anderen Endes (53) der zylinderförmigen Spritze (5) ausgebildet ist.

7. Applikator nach Anspruch 6, wobei
die vorbestimmten Winkelabstände Abstände von 60°, 90° oder 120° sind.

8. Applikator nach Anspruch 1, wobei
die Widerstandsstruktur (54, 75) benachbart in Bezug auf den Kartuscheinsetzabschnitt (52) der zylinderförmigen Spritze (5) bereitgestellt ist.

9. Applikator nach Anspruch 1, wobei
ein Anschlagsabschnitt (76) in der Nähe des Betätigungsabschnitts (74) des Stößels (7) bereitgestellt ist, um am anderen Ende (53) der zylinderförmigen Spritze (5) anzustoßen.

10. Applikator nach Anspruch 9, wobei
der Stößel (7) so geformt ist, dass die äußere Umfangsoberfläche eines Abschnitts (77), der benachbart in Bezug auf den Anschlagsabschnitt (76) vorliegt, in der Nähe der Innenumfangsoberfläche der zylinderförmigen Spritze (5) vorliegt.

11. Applikator nach Anspruch 2, wobei
die Widerstandsstruktur (54, 75) so angeordnet ist, dass die Kartusche (3) in den Kartuscheinsetzabschnitt (52) eingesetzt werden kann und die Kartusche (3) aus dem Kartuscheinsetzabschnitt (52) entfernt werden kann, wenn der zumindest eine innere vorstehende Abschnitt (75) des Stößels (7) in Kontakt mit dem zumindest einen äußeren vorstehenden Abschnitt (54) der zylinderförmigen Spritze (5) steht.

## Revendications

1. Applicateur comprenant :
une seringue cylindrique (5) moulée à partir d'un matériau en résine et ayant deux extrémités, la seringue cylindrique (5) comprenant une partie de montage de cartouche (52) au niveau de l'une (50) des deux extrémités de la seringue cylindrique (5), la partie de montage de cartouche (52) étant configurée pour être montée avec une cartouche (3) qui comprend une partie cylindrique (33) ayant deux extrémités et une partie d'ouverture (32) formée au niveau de l'une (31) des deux extrémités de la partie cylindrique (33), une partie de buse (35) prévue au niveau de l'autre (34) des deux extrémités de la partie cylindrique (33), et un piston (4) disposé à l'intérieur de la partie cylindrique (33) pour pousser un contenu, contenu dans la partie cylindrique (33) de la partie de buse (35) ; et
un piston plongeur (7) moulé à partir d'un matériau en résine et ayant deux extrémités, le piston plongeur (7) comprenant une partie de pression (72) prévue au niveau de l'une (71) des deux extrémités du piston plongeur (7) et configurée pour être insérée dans la seringue cylindrique (5) à partir de l'autre (53) des deux extrémités de la seringue cylindrique (5) et pour comprimer le piston (4) à l'intérieur de la cartouche (3) montée avec la partie de montage de cartouche (52), et une position actionnée (74) prévue au niveau de l'autre (73) des deux extrémités du piston plongeur (7), s'étendant à travers l'autre extrémité (53) de la seringue cylindrique (5), et configurée pour être enfoncée avec un doigt d'un opérateur, **caractérisé en ce que** :
l'applicateur comprend en outre une structure de résistance (54, 75) prévue entre une surface périphérique interne de la seringue cylindrique (5) et une surface périphérique externe de la partie de pression (72) du piston plongeur (7), la structure de résistance étant configurée pour servir de résistance au mouvement de la partie de pression (72) lorsque la partie de pression (72) est déplacée vers la partie de montage de cartouche (52) au-delà d'une position (P) prédéterminée et au mouvement de la partie de pression (72) lorsque la partie de pression (72) est déplacée du côté de la partie de montage de cartouche (52) vers l'autre extrémité (53) de la seringue cylindrique (5) au-delà de la position (P) prédéterminée, dans lequel :
la structure de résistance (54, 75) est configurée pour permettre à la partie de pression (72) d'être déplacée au-delà de la position (P) prédéterminée contre la résistance lorsqu'une force égale ou supérieure à une force prédéterminée est appliquée sur le piston plongeur (7) dans une direction longitudinale du piston plongeur (7).

2. Applicateur selon la revendication 1, dans lequel :
la structure de résistance (54, 75) comprend au moins une partie en saillie externe (54) formée de manière solidaire avec la surface périphérique interne de la seringue cylindrique (5) pour faire saillie radialement vers l'intérieur et au moins une partie en saillie interne (75) formée de manière solidaire avec la surface périphérique externe de la partie de pression (72) du piston plongeur (7) pour faire saillie radialement vers l'extérieur ; et
le matériau en résine pour former la seringue cylindrique (5) et une forme de la au moins une partie en saillie externe (54) et le matériau en résine pour former le piston plongeur (7) et une forme de la au moins une partie en saillie interne (75) sont déterminés de sorte que la au moins une partie en saillie interne (75) et la au moins une partie en saillie externe (54) sont déformées pour permettre à la au moins une partie en saillie interne (75) de passer sur la au moins une partie en saillie externe (54) lorsqu'une force égale ou supérieure à la force prédéterminée est appliquée sur le piston plongeur (7).

3. Applicateur selon la revendication 2, dans lequel :
le matériau en résine pour former la seringue cylindrique (5) et le matériau en résine pour former le piston plongeur (7) sont du polypropylène.

4. Applicateur selon la revendication 2 ou 3, dans lequel :
la au moins une partie en saillie interne (75) est constituée d'une partie en saillie annulaire (75') qui s'étend de manière continue et annulaire sur la surface périphérique externe du piston plongeur (7), et une forme transversale de la partie en saillie annulaire (75') a un profilé dont la hauteur augmente progressivement de manière continue vers un sommet du profilé, lorsque la partie en saillie annulaire (75') est coupée dans une direction orthogonale à une direction circonférentielle du piston plongeur (7) ; et
la au moins une partie en saillie externe (54) est constituée à partir d'une pluralité de parties en saillie (54') réparties, prévues à intervalles angulaires prédéterminés dans une direction circonférentielle de la seringue cylindrique (5), et une forme transversale de chaque partie en saillie (54') répartie a un profilé dont la hauteur augmente progressivement de manière continue vers un sommet du profilé, lorsque les parties en saillie (54') réparties sont coupées dans une direction orthogonale à la direction circonférentielle de la seringue cylindrique (5) et une forme transversale de chaque partie en saillie (54') répartie a un profilé dont la hauteur augmente progressivement de manière continue vers un sommet du profilé, lorsque les parties en saillie (54') réparties sont coupées dans la direction circonférentielle de la seringue cylindrique (5).

5. Applicateur selon la revendication 2 ou 3, dans lequel :
la au moins une partie en saillie externe est constituée à partir d'une partie en saillie annulaire qui s'étend de manière continue et annulaire sur la surface périphérique interne de la seringue cylindrique (5), et une forme transversale de la partie en saillie annulaire a un profilé dont la hauteur augmente progressivement de manière continue vers un sommet du profilé, lorsque la partie en saillie annulaire est coupée dans une direction orthogonale à une direction circonférentielle de la seringue cylindrique (5) ; et
la au moins une partie en saillie interne est constituée à partir d'une pluralité de parties en saillie réparties prévues à des intervalles angulaires prédéterminés dans une direction circonférentielle du piston plongeur (7), et une forme transversale de chaque partie en saillie répartie a un profilé dont la hauteur augmente progressivement de manière continue vers un sommet du profilé, lorsque les parties en saillie réparties sont coupées dans la direction orthogonale à la direction circonférentielle du piston plongeur (7) et une forme transversale de chaque partie en saillie répartie a un profilé dont la hauteur augmente progressivement de manière continue vers un sommet du profilé, lorsque les parties en saillie réparties sont coupées dans la direction circonférentielle du piston plongeur (7).

6. Applicateur selon la revendication 4 ou 5, dans lequel :
le profilé de chaque partie en saillie (54') répartie a une partie plate (54'A) prévue au sommet du profilé, et une jupe (54'C) formée sur le côté de la partie de montage de cartouche (52) par rapport à la partie plate (54'A) est plus longue qu'une jupe (54'B) formée sur le côté de l'autre extrémité (53) de la seringue cylindrique (5) par rapport à la partie plate (54'A).

7. Applicateur selon la revendication 6, dans lequel :
les intervalles angulaires prédéterminés sont des intervalles de 60°, 90° ou 120°.

8. Applicateur selon la revendication 1, dans lequel :
la structure de résistance (54, 75) est prévue de manière adjacente à la partie de montage de cartouche (52) de la seringue cylindrique (5).

9. Applicateur selon la revendication 1, dans lequel :
une partie de butée (76) est prévue à proximité de la partie actionnée (74) du piston plongeur (7) pour venir en butée contre l'autre extrémité (53) de la seringue cylindrique (5).

10. Applicateur selon la revendication 9, dans lequel :
le piston plongeur (7) est formé de sorte que la surface périphérique externe d'une partie (77) qui est adjacente à la partie de butée (76), est à proximité de la surface périphérique interne de la seringue cylindrique (5).

11. Applicateur selon la revendication 2, dans lequel :
la structure de résistance (54, 75) est positionnée de sorte que la cartouche (3) peut être montée avec la partie de montage de cartouche (52) et la cartouche (3) peut être retirée de la partie de montage de cartouche (52) lorsque la au moins une partie en saillie interne (75) du piston plongeur (7) est en contact avec la au moins une partie en saillie externe (54) de la seringue cylindrique (5).
